# EUROPEAN PATENT APPLICATION

(11) **EP 1 845 373 A1**
(43) Date of publication of application: **17.10.2007**
(21) Application number: 06007803.7
(22) Date of filing: 13.04.2006
(51) Int. Cl.: G01N 33/50, G01N 33/543

(54) **An immunoassay method**

(71) Applicant: Olympus Life and Material Science Europa GmbH, 20097 Hamburg (DE)
(72) Inventor: Gunzer, Gerhard, Dr., Limerick Road Ennis, Co. Clare (IE); Cavalleri, Marco, Tulla, Co. Clare (IE); McCusker, Matthew, Tulla Road, Ennis, Co. Clare (IE)
(74) Representative: Emmel, Thomas

(57) **Abstract**

An immunoassay method comprising the steps of: preparing a measuring sample by mixing a specimen with an immunoreactive substance, capable of specifically binding to a defined analyte, allowing the sample to react for a given reaction time, measuring a signal related to interactions of the immunoreactive substance with the analyte in the sample over the reaction time, checking on basis of signals measured at different times, whether or not prozone effect has occurred in the sample and marking of a prozone-positive sample accordingly, and calculating from at least one of the signals measured during the reaction time the concentration of analyte in the sample by using a calibration curve or a portion of a calibration curve covering concentrations of analyte causing prozone effect for prozone positive samples, and another calibration curve or portion or calibration curve covering concentrations of analyte not causing prozone effect for the remaining samples.

## Description

The invention relates to a method for an immunoassay in which an immunoreactive substance, usually an antibody, is added to a specimen in order to detect and/or quantify a defined analyte possibly contained in the specimen.

In most cases the immunoreactive substance used in immunoassay methods is a specific antibody. However, it is also possible to use antigens if antibodies are the analytes of interest.

In both cases the immunoreactive substance used is capable of specifically interacting with the analyte in question by e.g. forming antibody-antigen complexes. The formation of such complexes (agglutination) in a sample can be monitored by e.g. turbidimetric measurement over a given time. The degree of e.g. agglutination measured by turbidimetric assay is an indication on the concentration of analyte in the sample.

Usually a calibration curve or the like is established prior to analysis and the concentration of analyte is calculated by means of this curve using the values obtained during turbidimetric measurement.

Occasionally one observes in samples containing high concentrations of analyte, that no or only reduced agglutination occurs. If such samples are sufficiently diluted, normal agglutination will occur.

The lack or reduction of agglutination at high analyte-concentrations is called prozone effect or "hook effect" and is related to the fact that e.g. excess of an antigen in a sample results in formation of small antibody-antigen-complexes which do not clump to form visible agglutination. In general one can say that the higher the concentration of an antigen is in comparison to that of an antibody, the lower is the signal observed.

As increasing concentration of antigen exceeds the relative capability of the population of antibodies to recognise and bind, the signal decreases. This effect is dynamic and represents the change in reaction equilibrium from antigen-antibody aggregate formation to a higher probability of non-aggregated forms predominating.

Known immunoassay methods provide different approaches to identify samples in which prozone effect occurs. Most of the known methods use turbidimetric measurement of a sample over a given time to establish a reaction kinetic. From the reaction kinetic, e.g. by comparing values obtained at different times, it is possible to deduct whether or not prozone effect has occurred in a sample.

Methods to detect the prozone effect are e.g. disclosed in JP 2000221195, JP 6213893 or JP 6094717, to give only a few examples.

At present the protocols of immunoassay methods provide that a sample, in which the prozone effect is positively detected (prozone-positive sample), is diluted and that a new analysis is performed with the diluted sample. However, as the concentration of analyte is not known, usually dilution series must be run in order to find the correct dilution ratio for a given sample. Such dilution series are disadvantageous, as they require time for running the test and analysing the result, space in the analysing machine and additional reagents and sample material.

It is therefore the object of the present invention to provide a method which simplifies examination of prozone-positive samples.

The object is realised by a method according to claim 1.

Like known methods also the method of the invention comprises the steps of :
- preparing a measuring sample by mixing a specimen with an immunoreactive substance, especially an antibody, capable of specifically binding to an analyte,
- allowing the sample to react for a given reaction time,
- measuring a signal related to the interaction of the immunoreactive substance with the analyte over the reaction time e.g. by turbidimetric assay, and
- checking on basis of signals measured at different times, whether or not prozone effect has occurred in the sample and marking of a prozone-positive sample accordingly.

In contrast to known methods the invention provides that different calibration curves or different portions of a single calibration curve are used to calculate the analyte concentration depending on whether or not the sample is prozone-positive.

Compared to known techniques which use only one calibration curve, the method according to the invention uses two different calibration curves or systems, one for prozone-positive samples and the other one for the remaining samples.

Surprisingly it turned out that e.g. turbidimetric measurement of samples containing high concentrations of analyte causing prozone effect, still reproducibly yields concentration dependent values for different concentrations in the prozone range.

Consequently it was possible to establish specific calibration curves which allow at least an approximate calculation of analyte concentration in prozone-positive samples.

The concentration values obtained by the method according to the invention for prozone positive samples can be either taken directly or be used to calculate the correct dilution ratio.

Optimally the method according to the invention avoids re-examination of prozone positive samples, e.g. in cases in which approximate results still meet the medical requirements of the test. If re-examination is considered necessary the concentration values obtained for prozone positive samples are so precise, that a correct dilution of the sample to a normal concentration range will be possible without performing dilution series.

The method of the invention only requires that e.g. the correct calibration curve is selected for calculation of analyte concentration. This is managed through analyzing the kinetics of the reaction. This makes analyses much easier and quicker.

The immunoassay method of the invention works with all known agglutination or haem-agglutination tests with and without particle enhancement.

Preferably the method of the invention uses turbidimetric techniques for determining interaction between e.g. antibodies and antigen. However, also other techniques which allow quantification of said interactions and which suffer from the prozone effect, can be used, like e.g. chemiluminescence, enzyme immunoassays, fluorescence or nephelometry to give only some examples. For that reason, even if herein it is mainly referred to turbidimetric measurement, the invention shall not be limited to the use of this technique.

Typical specimens which can be analysed by the method according to the invention are e.g. blood, serum or urine. In general any body liquid or tissue extract shall be encompassed by the term specimen which can be examined in an immunoassay.

Typical reaction times vary between 30 seconds and 10 minutes.

Turbidimetric assays can be performed by using a spectrophotometer or turbidimeter. The used wavelength depends on the assay. In general agglutination can be best measured in a range between 340 nm and 800nm.

Any known method to detect prozone in samples and to mark the samples accordingly can be used in the invention.

The method of the invention is especially advantageous if used in automatic analysers capable of detecting prozone effects with samples. The analyser can be programmed to switch over from one calibration curve to the other one, if it e.g. gets the information that a sample is prozone-positive.

In a further embodiment of the invention it can be provided that the immunoreactive substance is immobilized on micro particles, especially e.g. on polymeric beads like e.g. latex beads. However, any particle is suited which can be used in common immunoassays or micro particle enhanced light scattering agglutination assays, respectively. The advantage compared to direct agglutination is that by using immunoreactive substance coupled to micro particles, the sensitivity of the assay is increased.

Indeed, any immunoassay detection methodology exhibiting "hook effect" reaction kinetics can be adapted using any software-mediated flagging process similar to that described herein. This includes the greater majority of non-competitive immunoassays, some of which have been detected using neural network classifier systems (Ref Clin Chem Lab Med. 1999 Apr;37(4):471-6).

A further embodiment provides that once it is established that a sample is prozone positive, further processing of the sample is performed at a different setting.

E.g. it can be provided, that turbidimetric measurement of a prozone-positive sample is performed at a wavelength different from the wavelength used for a normal sample. As stated above, prozoning in a sample is caused by decrease of the quantity of the complexes formed due to increased likelihood of disassociated aggregates predominating. The change of wavelength can be performed already during the measuring step or in a subsequent measurement. In certain circumstances, the observed phenomenon mimics prozoning of a sample in the test, but is actually caused by an increase of the size of the complexes formed. As these complexes may not interfere appropriately at the measurement wavelength due to suboptimal Debeye or Mie-like light scattering effects, the observed effect is similar as if this was caused by decrease of the size of the complexes formed. Therefore, if the wavelength is also increased, a more quantitative measurement of the signal is possible under these conditions.

Beside the described change of wavelength it is also possible to use different reaction times or incubation temperatures for prozone positive samples.

In the following the invention shall be illustrated by means of an example referring to the following figures.
- Fig. 1: is showing a normal calibration curve,
- Fig. 2: is showing a prozone calibration curve,
- Fig. 3: is showing the comparison of concentration values calculated by the normal calibration curve and values recalculated (framed portion) by the prozone calibration curve.

### Example:

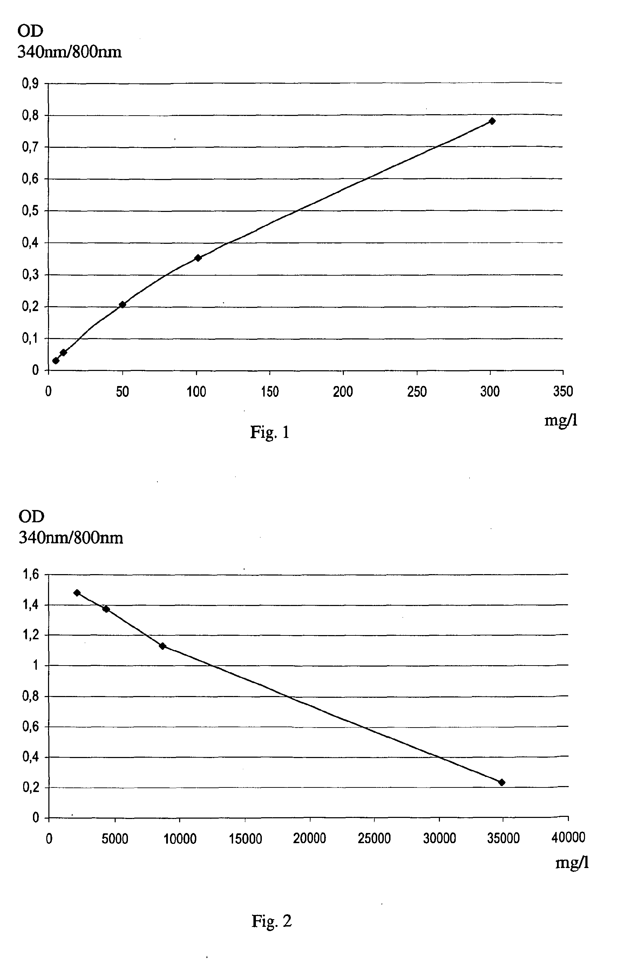

Two calibration curves were generated. The normal calibration curve was generated with Microalbumin material with concentrations covering the linear range (see Fig 1). A second calibration curve was generated using material at concentrations in the prozone region of the assay. This second curve was used as the "prozone calibration curve" (see Fig. 2). In Fig. 1 and Fig. 2 the bottom lines indicate the different concentrations (in mg/l) of the samples used for calibration. On the left side the OD (Optical Density)-signals taken at a wavelength of 340nm and secondary wavelength of 800nm are indicated.

To show reliability of the method according to the invention, immunoassays were performed with specimen containing different concentrations of Microalbumin (urinary Human Albumin). As immunoreactive substance a Goat anti-human Albumin antibody at an approximate concentration of 0.5mg/l was used.

The specimen was mixed with the antibody and turbidimetric measurement of the samples was performed over approximately 5 minutes at a primary wave length of 340nm and secondary wavelength of 800nm.

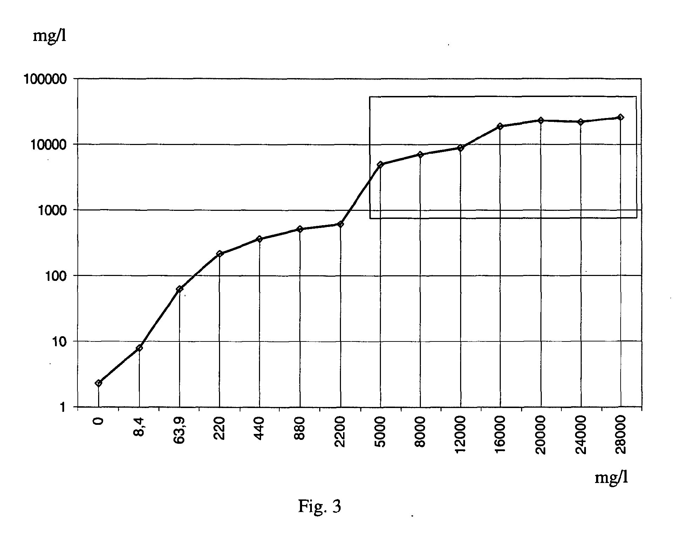

The results are shown in Fig. 3. The bottom line indicates the different concentrations (in mg/l) of microalbumin in the specimen assayed. On the left side is indicated the actual recovery calculated using the normal and prozone calibration curve.

The first portion of the curve in Fig. 3 (between 0-2200mg/l) represents concentration values which can be obtained by calculating OD-values of turbidimetric measurement with a normal calibration curve according to Fig. 1. This curve shows a linear range between 5 and 300 mg/l, representing the normal measuring range in which reliable results can be obtained. Above 300 mg/l, reliable calculation of values is not any more possible.

Starting from approximately a sample concentration of 2200 mg/l the samples begin to show prozone effect. For these samples the prozone calibration curve shown in Fig. 2 was used to recalculate the results. The recalculated results are shown by the framed portion of the curve in Fig. 3. One can see that using the prozone calibration curve to recalculate the results, concentration values are obtained which still approximately correspond to the concentration of the sample. At least the recalculated results are so close to the actual sample concentration that a correct dilution down to a concentration between 5 and 300 mg/l will be possible without running dilution series.

The effect of the prozone calibration range is to practically extend the range of this microalbumin assay by a factor of at least 85 (300mg/l to 26000mg/l).

## Claims

1. An immunoassay method comprising the steps of:
(a) preparing a calibration curve including different portions or preparing different calibration curves, with one portion or one calibration curve respectively covering concentrations of analyte not causing prozone effect and another portion or calibration curve covering concentrations of analyte causing prozone effect
(b) preparing a measuring sample by mixing a specimen with an immunoreactive substance, especially antibodies, capable of specifically binding to a defined analyte,
(c) allowing the sample to react for a given reaction time,
(d) measuring a signal related to interactions of the immunoreactive substance with the analyte in the sample over the reaction time
(e) checking on basis of signals measured at different times in step d, whether or not prozone effect has occurred in the sample and marking of a prozone-positive sample accordingly, and
(f) calculating from at least one of the signals measured in step d, preferably from the value taken at the end of the reaction time, the concentration of analyte in the sample by using the appropriate curve or portion of a curve generated in step a, with one curve or portion of the curve being used for prozone-positive samples and the other one for the remaining samples.

2. Method according to claim 1, **characterized in that** at least some of the steps a to f are performed by an automatic analyser.

3. Method according to claim 1, **characterized in that** interaction between immunoreactive substance and analyte is detected turbidimetrically.

4. Method according to claim 1, **characterized in that** the signal in step (d) is an optical signal which can be measured photometrically, especially turbidimetrically.

5. Method according to claim 1, **characterized in that** the antibodies or antigens used are bound to particles, especially polymeric beads.

6. Method according to claim 4, **characterized in that** depending on whether a sample is prozone-positive or not, the method is performed at different conditions.
